# FASCICULE DE BREVET EUROPEEN

(11) **EP 2 964 771 B1**
(45) Date de publication et mention de la délivrance du brevet: **26.06.2019**
(21) Numéro de dépôt: 14706897.7
(22) Date de dépôt: 06.02.2014
(51) Int. Cl.: C12P 7/10, C12P 7/14

(54) **PROCÉDÉ DE PRODUCTION D'ALCOOLS ET/OU DE SOLVANTS À PARTIR DE BIOMASSE LIGNOCELLULOSIQUE AVEC LAVAGE DU RESIDU SOLIDE OBTENU APRÈS FERMENTATION**
VERFAHREN ZUR HERSTELLUNG VON ALKOHOL UND / ODER LÖSUNGSMITTEL AUS BIOMASSE DURCH WASCHEN DES LIGNOCELLULOSEHALTIGEN, FESTEN RÜCKSTANDS DER NACH DER FERMENTATION ERHALTEN WIRD
PROCESS FOR PRODUCTION OF ALCOHOL AND / OR SOLVENTS FROM BIOMASS WITH WASHING OF THE LIGNOCELLULOSIC SOLID RESIDUE OBTAINED AFTER FERMENTATION

(30) Priorité: 06.03.2013 FR 1351989
(43) Date de publication de la demande: 13.01.2016
(73) Titulaire: IFP Energies nouvelles, 92500 Rueil-Malmaison (FR); L'Institut National de la Recherche Agronomique, 75338 Paris, Cedex 07 (FR); Agro Industrie Recherches et Développements, 51110 Pomacle (FR)
(72) Inventeur: AYMARD, Caroline, F-69002 Lyon (FR); BOUILLON, Pierre-Antoine, F-69003 Lyon (FR); FLEURIER, Stéphanie, F-69007 Lyon (FR); LOURET, Sylvain, F-69003 Lyon (FR); PEROTTA, Larissa, F-69007 Lyon (FR); TOTH, Eszter, F-69008 Lyon (FR)
(74) Mandataire: IFP Energies nouvelles
(86) Numéro de dépôt international: PCT/FR2014/050223
(87) Numéro de publication internationale: WO 2014/135755

(56) Documents cités:
- EP-A1- 2 169 074
- WO-A1-2009/015481
- WO-A1-2012/129622
- WO-A2-2010/130888
- US-A1- 2008 299 629
- EVA PALMQVIST ET AL: "Simultaneous detoxification and enzyme production of hemicellulose hydrolysates obtained after steam pretreatment", ENZYME AND MICROBIAL TECHNOLOGY, STONEHAM, MA, US, vol. 20, 1 janvier 1997 (1997-01-01), pages 286-293, XP007904791, ISSN: 0141-0229, DOI: 10.1016/S0141-0229(96)00130-5

## Description

La présente invention s'inscrit dans le cadre d'un procédé de production d'alcools et/ou de solvants dit de "seconde génération" à partir de biomasse lignocellulosique. Elle concerne plus particulièrement un procédé de production d'éthanol et/ou de solvants.

La biomasse lignocellulosique représente une des ressources renouvelables les plus abondantes sur terre. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).

La biomasse lignocellulosique est composée de trois principaux polymères : la cellulose (35 à 50%), qui est un polysaccharide essentiellement constitué d'hexoses ; l'hémicellulose (20 à 30%), qui est un polysaccharide essentiellement constitué de pentoses ; et la lignine (15 à 25%), qui est un polymère de structure complexe et de haut poids moléculaire, composé d'alcools aromatiques reliés par des liaisons éther. Ces différentes molécules sont responsables des propriétés intrinsèques de la paroi végétale et s'organisent en un enchevêtrement complexe.

Le procédé de transformation biochimique des matériaux lignocellulosiques en éthanol comprend en général une étape de prétraitement physico-chimique, suivie d'une étape d'hydrolyse enzymatique utilisant un cocktail enzymatique, d'une étape de fermentation éthanolique des sucres libérés et d'une étape de purification des produits de fermentation. Sous certaines configurations du schéma de procédé, les étapes d'hydrolyse enzymatique et de fermentation peuvent avoir lieu dans un même réacteur, dans une configuration de fermentation appelée SSF (Simultaneous Saccharification and Fermentation). Le schéma qui présente ces deux étape du procédé séparées est un schéma du type SHF (Separated Hydrolysys and Fermentation). Des exemples sont donnés par le document "Ethanol from lignocellulosics: A review of the economy", M. von Silvers et G. Zacchi, Bioresource Technology 56 (1996) 131-140.

Parmi les trois polymères de base qui intègrent la biomasse lignocellulosique, la cellulose et l'hémicellulose sont ceux qui peuvent le plus facilement être valorisés en produits de fermentation. La lignine reste inerte dans la plus grande partie des procédés. C'est pourquoi les procédés de production d'alcool et de solvant ont intérêt à séparer la lignine des mélanges réactionnels au plus tôt, afin de réduire la taille des unités et les coûts de traitement et investissement. La lignine peut être séparée à différentes étapes du procédé, par exemple au prétraitement, entre les étapes d'hydrolyse enzymatique et de fermentation, si le procédé est dans une configuration du tyoe SHF, ou dans l'étape de purification des produits de fermentation.

Le document "Fuel ethanol production : Process design trends and integration opportunities", C.A. Cardona et O. J. Sanchez, Bioresource Technology 98 (2007) 2415-2457, décrit un procédé où la lignine est solubilisée en présence d'un solvant permettant de séparer la lignine de la cellulose et de l'hémicellulose, la lignine étant ensuite précipitée pendant le prétraitement de la biomasse. Cette solution évite la présence de solides inertes dans le procédé dès l'étape d'hydrolyse enzymatique, mais présente des coûts opératoires élevés. De plus, cette configuration de procédé ne favorise pas une possible co-fermentation des sucres cellulosiques et hémicellulosiques récupérés au prétraitement, vu que deux flux présentant des propriétés et compositions distinctes sont obtenus.

Plusieurs procédés décrivent la séparation de la lignine entre les étapes d'hydrolyse enzymatique et de fermentation. Le document "A techno-economical comparison of three processes for the production of ethanol from pine", M. von Silvers et G. Zacchi, Bioresource Technology 51 (1995) 46-52, décrit l'impact du lavage du solide récupéré en termes de coût d'équipement de fermentation, ainsi qu'une baisse du titre éthanolique obtenu en fin de fermentation. Un avantage de lié à ce mode de production est la réduction de la taille des réacteurs grâce à l'élimination des solides inertes et la possibilité subséquente de séparation et recycle des microorganismes utilisés dans la fermentation vers les réacteurs de fermentation.

Néanmoins, le mode de production SSF est souvent décrit comme le plus avantageux en termes de rendement global. L'étape d'hydrolyse enzymatique seule est souvent pénalisée par la haute concentration de sucres dans le milieu en fin de réaction, vu que les sucres ont un effet inhibiteur sur l'activité des enzymes. Dans une configuration de procédé du type SSF, les microorganismes de fermentation consomment le sucre disponible dans le milieu, alors qu'en même temps le sucre est produit par solubilisation de la cellulose par les enzymes. La concentration de sucres dans le réacteur est ainsi toujours minimale et le rendement des deux réactions est maximal.

Des schémas de procédé basés sur la SSF n'ayant pas solubilisé la lignine en étape de prétraitement sont configurés pour séparer la lignine avant l'étape de purification des produits de fermentation afin d'éviter l'envoi de solides dans les colonnes de distillation en aval du procédé ("Ethanol from lignocellulosic biomass: technology, economics, and opportunities", C.E. Wyman, Bioresource Technology 50 (1994) 3-16).

Cependant, cette séparation est généralement réalisée par des outils de séparation physique, qui présentent le désavantage que la matière solide récupérée peut contenir encore des produits de fermentation piégés. Dans ce cas, il est possible d'ajouter une étape de lavage des solides au procédé, l'effluent de cette étape de lavage étant envoyé en étape de purification des produits de fermentation avec le vin de fermentation clarifié. Ceci représente un compromis entre la récupération des produits de fermentation et les limitations apportées par la pénalité énergétique liée à la dilution du vin à distiller.

La présente invention propose d'effectuer la séparation de la lignine et d'autres éventuels solides inertes après l'étape de fermentation. La matière solide composé majoritairement de lignine est ensuite soumis à un lavage pour récupérer les produits de fermentation piégés, en particulier les alcools et les solvants. Le liquide de lavage est ensuite recyclé dans l'unité d'hydrolyse enzymatique, qui peut être la même unité que l'unité de fermentation ou qui peut être distincte de l'unité de fermentation afin de ne pas apporter de la dilution aux flux existants.

De manière générale, l'invention a pour objet un procédé de production d'alcool et/ou de solvant à partir d'une charge de biomasse, dans lequel on effectue les étapes suivantes :
a) on effectue une étape de prétraitement par mise en contact et chauffage de la charge de biomasse avec de l'eau et un composé acide ou basique, de manière à obtenir un substrat prétraité,
b) on met en contact le substrat prétraité avec des enzymes cellulases et avec un flux liquide enrichi en produits de fermentation obtenu à l'étape e) de manière à obtenir un hydrolysat comportant un résidu solide et une phase liquide contenant des sucres,
c) on effectue une fermentation alcoolique de l'hydrolysat au moyen d'un microorganisme alcooligène de manière à produire un vin de fermentation comportant une matière solide et une phase liquide contenant des produits de fermentation,
d) on extrait au moins une partie de la matière solide contenue dans le vin de fermentation de manière à obtenir un flux enrichie en matière solide et un vin de fermentation appauvri en matière solide,
e) on lave le flux enrichi en matière solide avec un flux liquide de manière à obtenir ledit flux liquide enrichi en produits de fermentation, le flux liquide enrichi en produits de fermentation étant recyclé à l'étape b),
f) on effectue une étape de séparation du vin de fermentation appauvri en matière solide de manière à obtenir au moins un flux purifié comportant un alcool ou un solvant et au moins un flux de vinasse.

Selon l'invention, le flux liquide de l'étape e) peut être composé d'eau fraîche. Le flux liquide de l'étape e) peut comporter au moins une partie du flux de vinasse obtenu à l'étape f).

A l'étape b), le flux liquide enrichi en produits de fermentation peut avoir un débit compris entre 50% et 1500% poids du débit de substrat prétraité.

A l'étape e), on peut mettre en contact le flux enrichi en matière solide avec ledit flux liquide, puis on peut séparer le flux liquide de la matière solide.

On peut réaliser l'étape d) de manière à ce que ledit flux enrichi matière solide comporte entre 15% poids et 55% poids de matière solide et de manière à ce que ledit vin de fermentation appauvri en matière solide comporte moins de 15% poids de matière solide. On peut effectuer l'étape b) dans un premier réacteur et on peut effectuer l'étape c) dans un deuxième réacteur distinct du premier réacteur. Alternativement, on peut effectuer simultanément l'étape b) et l'étape c) dans le même réacteur.

Les enzymes cellulases peuvent être produites par un microorganisme choisi parmi les champignons appartenant aux genres *Trichoderma, Aspergillus, Penicillium ou Schizophyllum*, ou les bactéries anaérobies appartenant au genre *Clostridium.*

Le microorganisme alcooligène peut être choisi parmi les microorganismes du genre *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis, Zymomonas mobilis, Clostridium, Escherichia coli.*

La charge de biomasse peut être composée par au moins l'un des éléments suivants : du bois, des plantes cultivés, des déchets lignocellulosiques agricoles, des résidus de l'industrie de transformation des matériaux lignocellulosiques.

A l'étape a), on peut effectuer une explosion à la vapeur de la biomasse en comprimant, puis en effectuant une détente de la biomasse en mélange avec de l'eau et un composé acide.

A l'étape e), on peut mettre en oeuvre un organisme alcooligène produisant au moins de l'éthanol.

D'autres caractéristiques et avantages de l'invention seront mieux compris et apparaîtront clairement à la lecture de la description faite ci-après en se référant aux dessins parmi lesquels :
- la figure 1 est une représentation schématique d'un mode de réalisation non conforme à l'invention,
- la figure 2 est une représentation schématique d'un premier mode de réalisation du procédé suivant l'invention,
- la figure 3 est une représentation schématique d'un deuxième mode de réalisation du procédé selon l'invention.

Au sens de la présente invention, on désigne sous le terme de "matière sèche" les composés solides et solubles contenus dans un flux, le taux de matière sèche d'un flux est déterminé selon la méthode ASTM E1756-01, qui consiste en une perte de masse à 105°C. On désigne sous le terme "matière solide", les composés solides présents dans un flux, ces composés solides étant non soluble en phase liquide. La matière solide peut être composée de lignine, d'hémicellulose et/ou de cellulose. Le taux de matière solide contenue dans un flux peut être déterminé par lavages successifs du flux à l'eau et analyse de la teneur en matière sèche résiduelle du flux lavé.

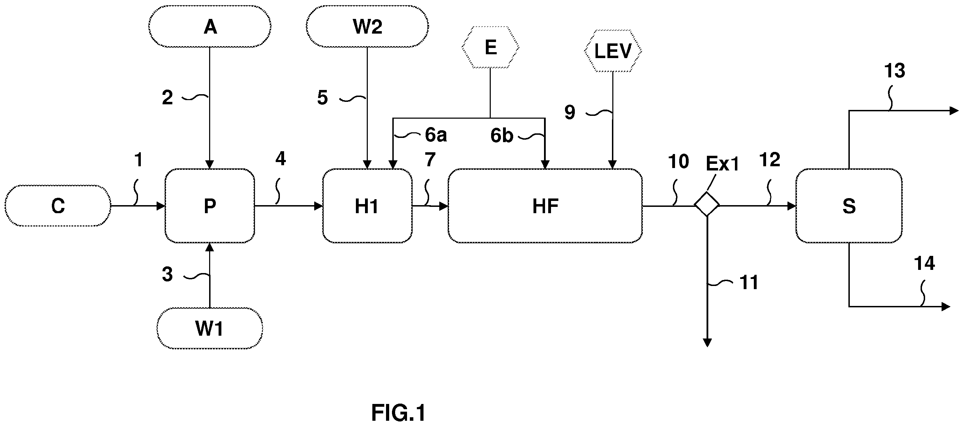

En référence à la figure 1 une charge de biomasse C est amenée dans l'unité de prétraitement P par l'intermédiaire du conduit 1. La charge de biomasse peut être composée de bois, des pailles ou de rafles de maïs, de produits de cultures forestières dédiées (par exemple de résineux tels les épicéas ou les pins, ou des feuillus tels les eucalyptus), des plantes de cultures dédiées tells le miscanthus ou le switchgrass, de résidus de plantes alcooligènes, sucrières (par exemple canne à sucre ou betterave) et céréalières (par exemple mais, blé...), de produits et résidus de l'industrie papetière et des produits de transformation des matériaux lignocellulosiques. La charge peut être composée d'environ 35 à 50 % poids de cellulose, de 20 à 30 % poids d'hémicellulose et de 15 à 25 % poids de lignine.

Le composé acide ou basique A et l'eau W1 nécessaires sont respectivement amenés dans l'unité de prétraitement P par l'intermédiaire des conduits 2 et 3 afin d'y réaliser une réaction d'hydrolyse en milieu acide ou basique. Dans l'unité P, la charge de biomasse C est mise en contact et mélangée avec l'eau W1 et le composé A dans un réacteur. L'unité P de prétraitement peut mettre en oeuvre une action mécanique, créée par exemple au moyen d'une extrudeuse de type bi-vis ou d'une défibreuse.

Parmi les composés acides, le composé A peut être choisi parmi de l'acide sulfurique, de l'acide chlorhydrique, de l'acide nitrique, de l'acide acétique ou de l'acide formique. Parmi les composés basiques, le composé A peut être choisi parmi de l'hydroxyde de potassium, de l'hydroxyde de sodium, de l'ammoniaque.

Lors de l'étape de prétraitement dans l'unité P, on effectue au moins une étape de chauffage du mélange de biomasse C, d'eau W1 et du composé A dans un réacteur. L'eau W1 peut être introduite sous forme de vapeur. Le rôle du prétraitement est de rendre la cellulose accessible aux enzymes en déstructurant la matrice lignocellulosique. Lors du prétraitement, on attaque préférentiellement l'hémicellulose, qui se retrouve en grande partie dissous dans la phase liquide.

Selon un premier mode de réalisation, on réalise un prétraitement alcalin dans l'unité P. Par exemple, dans l'unité P, on peut mettre en oeuvre un prétraitement au sulfate de sodium, encore appelé procédé Kraft, classiquement utilisé dans les procédés de production de produits papetiers, dit Kraft ou "pâte au sulfate", à l'issue duquel on obtient des pâtes papetières. Le prétraitement chimique alcalin réalisé dans l'unité P peut également être un prétraitement par explosion des fibres à l'ammoniac, encore appelé prétraitement AFEX (Ammonia Fiber Explosion) ou prétraitement par percolation utilisant de l'ammoniaque avec recycle, encore appelé prétraitement ARP (Ammonia Recycle Percolation).

Le procédé au sulfate de sodium ou procédé Kraft est basé sur l'utilisation de soude et de sulfate de sodium. Le traitement chimique des copeaux de bois se fait à 150-175°C pendant une durée de 1 à 7 heures en fonction du substrat utilisé. Les pâtes papetières kraft sont produites à partir des biomasses les plus variées, mais plus particulièrement depuis les espèces arborescentes résineuses (softwood tels que les épicéas ou les pins) ou feuillues (hardwood tels que les eucalyptus) ou encore les déchets lignocellulosiques agricoles (paille de blé, riz, etc.). Elles sont partiellement délignifiées au moyen de cuissons à haute température et en présence de soude. Cette délignification est contrôlée par les paramètres opératoires des réacteurs. La cuisson est réalisée dans un réacteur vertical, où les copeaux descendent par gravité et rencontrent les diverses liqueurs de cuisson. Le sulfure de sodium est préparé directement à partir de sulfate de sodium par combustion. Lors de la cuisson, le sulfure de sodium est hydrolysé en soude, en NaHS et en H₂S. Les différents composés soufrés présents réagissent avec la lignine pour donner des thiolignines plus facilement solubles. La liqueur appliquée aux copeaux est appelée liqueur blanche. La liqueur extraite du réacteur ou lessiveur contenant les composés éliminés de la paroi est appelée liqueur noire. A l'issue de ce prétraitement alcalin, on aboutit à la production d'un substrat prétraité, enrichi en cellulose puisqu'il contient entre 60 et 90% de cellulose et entre 5 et 20% d'hémicellulose.

Le procédé ARP (Ammonia Recycle Percolation) est un procédé de prétraitement utilisant de l'ammoniaque avec recyclage. Ce type de procédé est notamment décrit par Kim et al., 2003, Biores. Technol. 90 (2003) p 39-47. La température élevée de la percolation conduit à une solubilisation partielle à la fois de la lignine et des hémicelluloses, cette solution est ensuite chauffée pour recycler l'ammoniaque et récupérer d'une part la lignine extraite, par exemple pour une valorisation énergétique, et d'autre part les sucres solubles issus des hémicelluloses.

Le procédé AFEX (Ammonia Fiber Explosion) consiste à introduire le substrat lignocellulosique dans un cuiseur à haute pression en présence d'ammoniac, puis de provoquer une détente explosive en sortie du réacteur et de recycler l'ammoniac alors sous forme gazeuse. Ce type de procédé est notamment décrit par Teymouri et al., 2005, Biores. Technol. 96 (2005) p.2014-2018. Ce procédé conduit principalement à une déstructuration de la matrice de la biomasse mais il n'y a pas de séparation phasique des composés lignine, hémicellulose et cellulose en sortie de traitement.

Selon un deuxième mode de réalisation, on réalise un prétraitement acide dans l'unité P. Par exemple, dans l'unité P, on peut mettre en oeuvre un prétraitement de type cuisson à l'acide diluée. Dans ce mode de réalisation la biomasse est mise en contact avec un acide fort dilué dans de l'eau, par exemple l'acide sulfurique, en mettant en oeuvre la biomasse à de faibles teneurs en matières sèche, généralement compris entre 5 et 20% de matière sèche. La biomasse, l'acide et l'eau sont mis en contact dans un réacteur et monté en température, généralement entre 120°C et 200°C. Lors de ce procédé, les composés hémicellulosique sont principalement hydrolysés en sucres, permettant de déstructurer la matrice lignocellulosique. A l'issue de ce prétraitement acide, on aboutit à la production d'un substrat prétraité solide, enrichi en cellulose et en lignine ainsi qu'une fraction liquide enrichie en sucres.

Selon un troisième mode de réalisation on peut également mettre en oeuvre le procédé nommé "explosion vapeur", ou "SteamEx" ou "Steam Explosion" selon la terminologie anglo-saxonne, dans l'unité P. C'est un procédé dans lequel la biomasse lignocellulosique est mise en contact avec de l'eau dans un réacteur à faible temps de séjour, généralement compris entre 2 et 15 minutes et à des températures modérées, généralement entre 120°C et 250°C et à une pression comprise entre 5 et 50 bars. L'eau peut être additionné d'un composé acide, par exemple de l'acide sulfurique, ou d'un composé basique. En sortie de du réacteur, la biomasse est détendue, par exemple à pression atmosphérique, dans un récipient séparateur gaz / solide afin de produire une biomasse prétraitée à haute matière sèche, généralement comprise entre 20 et 70% de matière sèche.

L'unité P peut comporter des étapes additionnelles, par exemple de mise au pH, qui ont pour but de faciliter la mise en oeuvre et l'efficacité des étapes d'hydrolyse enzymatique et de fermentation.

On évacue de l'unité P un substrat prétraité par le conduit 4. Le substrat prétraité est composé de sucres dissous en phase liquide et de matière solide composée de lignine, de cellulose et d'hémicellulose qui n'a pas été liquéfié dans le prétraitement P. Le flux de substrat prétraité circulant dans le conduit 4 contient préférentiellement entre 10% poids et 60% poids de matière sèche et encore plus préférentiellement entre 20% poids et 55% poids de matière sèche. On évacue de l'unité P un substrat prétraité par le conduit 4 dans l'unité H1.

Le substrat prétraité est introduit dans un réacteur de l'unité H1 pour subir une première étape d'hydrolyse enzymatique dite de "liquéfaction". De l'eau W2 et des enzymes E sont respectivement ajoutés dans l'unité H par l'intermédiaire des conduits 5 et 6a afin de réaliser une réaction d'hydrolyse enzymatique du substrat prétraité. Les quantités de substrat prétraité, d'eau et d'enzyme sont ajustées dans l'étape dans l'unité H1 de manière à ce que le milieu réactionnel comporte une teneur en matière solide généralement comprises entre 5% et 40% poids, de préférence entre 10% et 25% poids. La liquéfaction est préférentiellement réalisée à pH compris entre 4 et 5,5 et à une température comprise entre 40°C et 60°C. Les enzymes E peuvent être produitespar un microorganisme, par exemples des champignons appartenant aux genres *Trichoderma, Aspergillus, Penicillium ou Schizophyllum,* ou les bactéries anaérobies appartenant par exemple au genre *Clostridium.* Les enzymes produites par ces microorganismes contiennent notamment les cellulases et, éventuellement, des hémicellulases, adaptées à l'hydrolyse poussée de la cellulose et, éventuellement, des hémicelluloses. Dans l'unité H1, les conditions de l'hydrolyse enzymatique, principalement le taux de matière sèche du mélange à hydrolyser et la quantité d'enzymes utilisée, sont choisies de telle façon que l'on obtienne une solubilisation de la cellulose comprise entre 10% et 40% poids, de préférence entre 20% et 40% poids, par au poids total de cellulose contenu dans le substrat prétraité. On évacue de l'unité H1 un substrat liquéfié par le conduit 7 pour l'introduire dans l'unité HF. Ainsi, le flux de substrat liquéfié issu de H1 comporte des sucres en dissous en phase aqueuse et de la matière solide composée principalement de lignine et riche en cellulose.

Ledit substrat liquéfié subit simultanément, dans le réacteur de l'unité HF, une hydrolyse et une fermentation. une hydrolyse complète afin de solubiliser tous les sucres présents dans la phase solide. dans le réacteur de l'unité HF, on peut ajouter des enzymes par du conduit 6b. De plus, dans l'unité HF, le substrat hydrolysé est mis en contact avec un ou plusieurs micro-organismes de fermentation LEV introduits par le conduit 9. Les microorganismes LEV peuvent être choisis par exemple parmi les éléments suivants : les levures du genre *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis* ou les bactéries du genre *Zymomonas mobilis, Clostridium, Escherichia coli.* De manière très préférée, on utilise une levure du type *Saccharomyces,* et encore plus préférentiellement *Saccharomyces cerevisiae.* De préférence, on utilise une levure adaptée à produire de l'éthanol, par exemple les levures... Les sucres fermentescibles sont ainsi transformés en alcools et/ou solvants par les micro-organismes. L'étape d'hydrolyse et de fermentation dans l'unité HF peut être réalisée à une température comprise entre 30°C et 35°C. Dans cette unité HF, la réaction de fermentation produit un vin de fermentation enrichi en produits de la réaction de fermentation, évacué par le conduit 10, par exemple des alcools ou des solvants organiques. Le vin de fermentation contient également de la matière solide composée principalement de lignine.

Ledit vin de fermentation est introduit dans l'unité Ex1 pour subir une étape de séparation entre liquide et solide afin d'en extraire la matière solide, notamment la lignine qui n'a pas été hydrolysée ni fermentée dans les unités H1 et HF. L'extraction de la matière solide est effectuée dans l'unité Ex1, qui peut mettre en oeuvre l'une des techniques suivantes : centrifugation, essorage ou pressage, filtration, décantation. L'unité Ex1 produit un flux appauvri en matière solide évacué par le conduit 12 et un flux enrichi en matière solide, notamment en lignine, évacué par le conduit 11.

Le flux appauvri en matière solide est ensuite introduit par le conduit 12 dans l'unité de séparation S afin d'en extraire les composés d'intérêt évacués par le conduit 13, par exemple des alcools ou des solvants organiques. Les résidus de la séparation, couramment appelés vinasses, sont évacués de l'unité de séparation S par le conduit 14. Les vinasses sont généralement composées d'eau ainsi que de tout produit liquide ou solide non converti ou non extrait lors des étapes précédentes dans les unités H1, HF et Ex1. L'unité de séparation S peut mettre en oeuvre une ou plusieurs distillations, et éventuellement une séparation des matières en suspension par exemple par centrifugation, décantation, filtration.

Le procédé non conforme à l'invention schématisé par la figure 1 présente l'inconvénient d'évacuer une partie des composés valorisés, c'est-à-dire des alcools ou des solvants, qui restent contenus dans la matière solide, c'est-à-dire essentiellement la lignine, évacuée par le conduit 11 lors de l'opération d'extraction de la matière solide dans l'unité Ex1. En effet, ces alcools ou solvants présents sous forme liquide dans le vin de fermentation circulant dans le conduit 10 risquent d'être séparés de façon imparfaite dans l'étape Ex1 avec les outils connus de l'homme de l'art, par exemple des outils de centrifugation, essorage, décantation ou pressage. Au moins une fraction des alcools ou solvants est évacuée par le conduit 11 provoquant une perte de rendement du procédé.

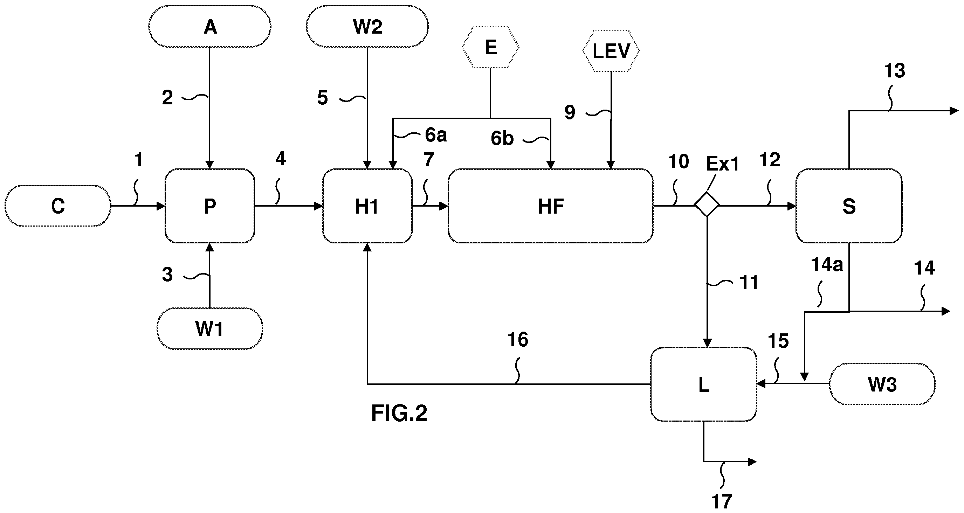

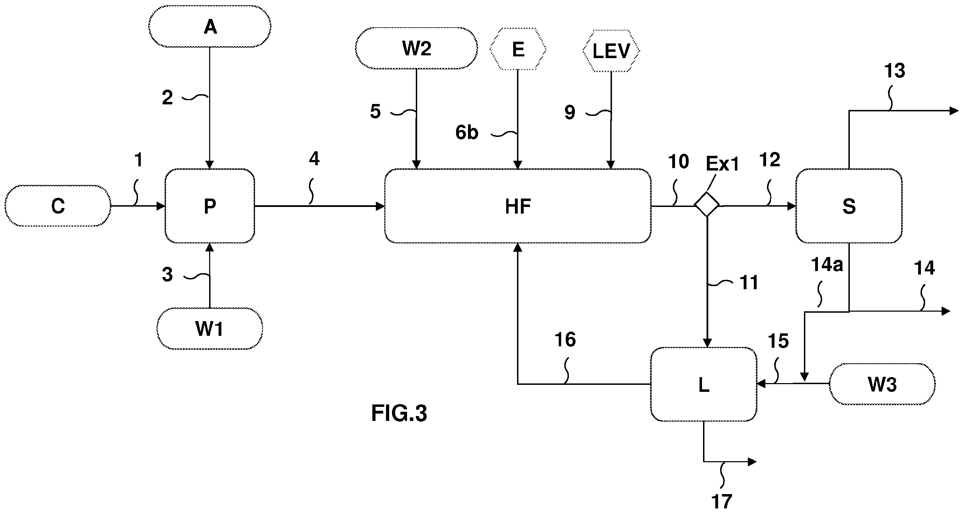

Le procédé selon l'invention propose de remédier à ce problème de perte de produits de fermentation dans la matière solide en mettant en oeuvre une opération de lavage du flux enrichi en matière solide circulant dans le conduit 11, permettant de recycler les produits de fermentation dans le procédé sans y apporter une dilution supplémentaire. L'invention sera mieux comprise à la lecture de la description des figures 2 et 3, schématisant deux mises en oeuvre du procédé selon l'invention. Les références de la figure 2 identiques à celles de la figure 1 désignent les mêmes éléments.

Selon l'invention, on peut effectuer l'étape de séparation de la matière solide du liquide dans l'unité Ex1 de manière à ce que le flux 8 appauvri en solide contienne moins de 15% poids, et de préférence moins de 10% poids, et encore plus préférentiellement moins de 5% poids de matières solide. Le reste du flux 8 peut être composé de sucre dissous en phase aqueuse. De plus, on peut effectuer l'étape de séparation de la matière solide du liquide dans l'unité Ex1 de manière à ce que le flux 9 enrichi en solides contienne entre 15% et 55% poids, et de préférence entre 20% et 45% poids et encore plus préférentiellement entre 25% et 35% poids de matière solide. Du fait des limitations des équipements de séparation entre solides et liquides de l'unité Ex1, le flux 9 enrichi en solides contient au moins 45% de liquide, qui peut notamment être composé de sucre dissous en phase aqueuse.

En référence à la figure 2, on effectue, dans l'unité L, une étape de lavage de la matière solide contenue dans le flux arrivant par le conduit 11. Dans l'unité L, un flux liquide est amené par l'intermédiaire du conduit 15 afin de réaliser un lavage de la matière solide contenue dans le flux arrivant par le conduit 11. Le flux liquide est mis en contact avec la matière solide, puis le liquide est séparé de la matière solide. L'étape de lavage dans l'unité L peut être réalisée par percolation, par opérations de mélange et séparation liquide/solide successives ou par toute autre technique connue de l'homme de l'art. Le lavage permet d'extraire par le conduit 16 un flux liquide enrichi en composés d'intérêt, c'est-à-dire des alcools ou des solvants, ainsi qu'un flux appauvri en composés d'intérêt par le conduit 17. Le flux 16 est ensuite recyclé dans l'unité H1, afin d'augmenter la récupération des produits de fermentation et accroître le rendement global du procédé.

En référence à la figure 2, le flux liquide amené par le conduit 15 peut être un flux d'eau fraiche W3 ou une portion des vinasses provenant de l'unité S amenée par les conduits 14 puis 14a dans l'unité L. Selon l'invention, le fait de recycler le flux enrichi en composés d'intérêt par le conduit 16 dans l'unité H1 permet de limiter, voire de supprimer, l'apport d'eau fraiche W2 directement dans l'unité H1. Par exemple le flux 16 représente entre 50%poids et 1500%poids, préférentiellement entre 100%poids et 600%poids, du débit de substrat prétraité introduit par le conduit 4 dans l'unité H1. Ainsi, la présente invention permet de limiter, voire d'éviter, toute dilution supplémentaire des flux dans le procédé liée à l'emploi d'eau pour le lavage du flux 11.

Dans le procédé schématisé par la figure 3, le substrat prétraité issu de l'unité P est directement introduit par le conduit 4 puis 7 dans le réacteur de l'unité HF d'hydrolyse et de fermentation simultanée. L'eau W2, les enzymes E et les levures LEV sont introduites dans le réacteur de l'unité HF respectivement par les conduit 5, 6b et 9. Dans ce cas, le flux 16 enrichi en composé d'intérêt est introduit dans le réacteur de l'unité HF.

### EXEMPLES

Les exemples ci-après illustrent l'invention, sans en limiter la portée.

### Exemple 1 selon le procédé de la figure 1 (non conforme à l'invention)

Dans cet exemple, on présente un procédé de production d'éthanol à partir de paille avec les caractéristiques suivantes:
Charge: Paille de blé, débit 89,60 tonne / heure, composition moyenne :

| | % (base sèche) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la matière sèche (MS) du solide en entrée de prétraitement est de 45% poids. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique H1.

### Hydrolyse enzymatique et conversion en éthanol :

La concentration en solides à l'entrée du réacteur de liquéfaction dans l'unité H1 est de 14% poids. Pour atteindre ce niveau de dilution, 114,3 tonnes / heure d'eau W2 de procédé sont mélangées au substrat prétraité. Après 10 heures d'opération, le mélange liquéfié est envoyé dans l"unité HF, qui opère en mode SSF. Dans cette étape, les enzymes continuent à agir sur la cellulose, en même temps que le microorganisme de fermentation (*Saccharomyces cerevisiae*) agit sur les sucres libérés. Le rendement global de la conversion de la cellulose en éthanol est de 0,40 g d'éthanol par g de cellulose introduite. Les pentoses présents ne sont pas convertis par le microorganisme de fermentation choisi. Le titre éthanolique du flux envoyé en séparation est de 39,4 g d'éthanol / kg de vin.

### Séparation :

Le vin issu de l'unités HF est envoyé dans une décanteuse dans l'unité Ex1, où la matière solide, notamment la lignine, est séparée du vin avant l'étape de récupération de l'éthanol dans l'unité de séparation S. Dans cette étape de séparation, un flux contenant 31,3% de matière solide est produit. Étant donnée la nature de la séparation, ce flux contient encore une quantité importante de produits de fermentation, notamment de l'éthanol, piégés. La perte d'éthanol lors de cette étape est estimée à **20,7%.**

### Récupération de l'éthanol :

La séparation dans l'unité S se fait par distillation. Le rendement d'extraction est de 99,6%. Ainsi, le procédé permet de produire annuellement **74 890 tonnes d'éthanol**, et a une consommation spécifique d'eau de procédé de **12,2 tonnes d'eau / tonne d'éthanol produit.**

### Exemple 2 selon le procédé de la figure 1 (non conforme à l'invention)

Dans cet exemple, on présente un procédé de production d'éthanol à partir de paille avec les caractéristiques suivantes:
Charge: Paille de blé, débit 89,60 tonne / heure, composition moyenne :

| | % (base sèche) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la matière sèche (MS) du solide en entrée de prétraitement est de 45% poids. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique H1.

### Hydrolyse enzymatique et conversion en éthanol :

La concentration en solides à l'entrée du réacteur de liquéfaction dans l'unité H1 est de 14%. Pour atteindre ce niveau de dilution, 114,3 tonnes / heure d'eau W2 de procédé sont mélangées au substrat prétraité. Après 10 heures d'opération, le mélange liquéfié est envoyé dans l'unité HF, qui opère en mode SSF. Dans cette étape, les enzymes continuent à agir sur la cellulose, en même temps que le microorganisme de fermentation (*Saccharomyces cerevisiae*) agit sur les sucres libérés. Le rendement global de la conversion de la cellulose en éthanol est de 0,40 g d'éthanol par g de cellulose introduite. Les pentoses présents ne sont pas convertis par le microorganisme de fermentation choisi. Le titre éthanolique du flux envoyé en séparation est de 39,4 g d'éthanol / kg de vin.

### Séparation :

Le vin issu de l'unité HF est envoyé dans une décanteuse dans l'unité Ex1, où la matière solide, notamment la lignine, est séparée du vin avant l'étape de récupération de l'éthanol dans l'unité de séparation S. Dans cette étape de séparation, un flux contenant 30,8% de matière solide est produit.

### Lavage du flux de matière solide :

Étant donnée la nature de la séparation, environ 20% de l'éthanol restent piégés dans la matière solide. Un lavage en contrecourant de la matière solide est alors mis en oeuvre dans le but de réduire les pertes. 115 tonnes / heure d'eau de procédé sont utilisées dans cette opération. Les pertes d'éthanol baissent à **1,4%.** Le vin dilué récupéré contient 20,1 g d'éthanol / kg de vin et est mélangé au vin clarifié obtenu avant d'être envoyé à l'unité de séparation S.

### Récupération de l'éthanol :

Le vin clarifié mélangé au vin dilué issu du lavage est envoyé vers les colonnes de distillation de l'unité S. Dû à la basse concentration en éthanol du vin dilué issu du lavage, le vin total ne contient que 35,2 g d'éthanol par kg de vin en entrée de l'étape de distillation, **valeur 10,7% inférieure à celle obtenu en fin de fermentation SSF.**
La séparation dans l'unité S se fait par distillation. Le rendement d'extraction est de 99,6%. Ainsi, le procédé permet de produire annuellement **93 087 tonnes d'éthanol**, et a une consommation spécifique d'eau de procédé de **19,6 tonnes d'eau / tonne d'éthanol produit.**

### Exemple 3 selon le procédé de la figure 2 (conforme à l'invention)

Dans cet exemple, on présente un procédé de production d'éthanol à partir de paille avec les caractéristiques suivantes:
Charge: Paille de blé, débit 89,60 tonne / heure, composition moyenne :

| | % (base sèche) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la matière sèche (MS) du solide en entrée de prétraitement est de 45% poids. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique H1.

### Hydrolyse enzymatique et conversion en éthanol :

La concentration en solides à l'entrée du réacteur de liquéfaction dans l'unité H1 est de 14% poids. Pour atteindre ce niveau de dilution, 114,3 tonnes / heure d'eau de dilution arrivant par le conduit 16 sont mélangées au substrat prétraité. Cette eau de dilution contient 25,0 g d'éthanol / kg de solution originaires du recyclage de l'eau utilisée pour le lavage du flux de solides obtenu à l'issue de l'opération de séparation liquide/solide Ex1 en sortie de l'unité H F.

Après 10 heures d'opération, le mélange liquéfié est envoyé dans l'unité HF, qui opère en mode SSF. Dans cette étape, les enzymes continuent à agir sur la cellulose, en même temps que le microorganisme de fermentation (*Saccharomyces cerevisiae*) agit sur les sucres libérés. Le rendement global de la conversion de la cellulose en éthanol est de 0,40 g d'éthanol par g de cellulose introduite. Les pentoses présents ne sont pas convertis par le microorganisme de fermentation choisi. Le titre éthanolique du flux envoyé en séparation est de 48,9 g d'éthanol / kg de vin.

### Séparation :

Le vin issu de l'unité HF est envoyé vers une décanteuse dans l'unité Ex1, où la matière solide, notamment la lignine, est séparée du vin avant l'étape de récupération de l'éthanol dans l'unité de séparation S. Dans cette étape de séparation, un flux contenant 30,7% poids de matière solide est produit.

### Lavage du flux de matière solide :

Afin de récupérer l'éthanol piégé dans la matière solide, une étape de lavage de la matière solide est mise en oeuvre. Dans cet exemple, la totalité de l'eau envoyée comme eau de dilution à l'étape d'hydrolyse enzymatique est d'abord utilisée pour laver la matière solide dans l'unité de lavage L à contrecourant sous trois étages de contact. Puis l'eau de lavage est introduite dans l'unité H1. Les pertes d'éthanol sont alors **réduites de 93,2%** par rapport aux résultats de l'exemple 1, soit **1,4%** de perte globale.

### Récupération de l'éthanol :

La séparation dans l'unité S se fait par distillation. Le rendement d'extraction est de 99,6%. Ainsi, le procédé permet de produire annuellement **92 607 tonnes d'éthanol**, et a une consommation spécifique d'eau de procédé de **9,9 tonnes d'eau / tonne d'éthanol produit,** soit 18,8% et 49,5% de réduction par rapport aux consommations d'eau dans les exemples 1 et 2, respectivement.

### Exemple 4 selon le procédé de la figure 1 (non conforme à l'invention)

Dans cet exemple, on présente un procédé de production d'éthanol à partir de paille avec les caractéristiques suivantes:
Charge: Paille de blé, débit 89,60 tonne / heure, composition moyenne :

| | % (base sèche) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la matière sèche (MS) du solide en entrée de prétraitement est de 45% poids. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique H1.

### Hydrolyse enzymatique et conversion en éthanol :

La concentration en solides à l'entrée du réacteur de liquéfaction dans l'unité H1 est de 14% poids. Pour atteindre ce niveau de dilution, 114,3 tonnes / heure d'eau W2 de procédé sont mélangées au substrat prétraité. Après 10 heures d'opération, le mélange liquéfié est envoyé dans l'unité HF, qui opère en mode SSF. Dans cette étape, les enzymes continuent à agir sur la cellulose, en même temps que le microorganisme de fermentation (*Saccharomyces cerevisiae* provenant d'une souche modifiée capable de réaliser la co-fermentation des hexoses et des pentoses) agit sur les pentoses présents et les hexoses libérés. Le rendement global de la conversion de la cellulose en éthanol est de 0,39 g d'éthanol par g de cellulose introduite. La conversion des pentoses en éthanol s'élève à 0,11 g d'éthanol par g de pentose introduite. Le titre éthanolique du flux envoyé en séparation est de 47,5 g d'éthanol / kg de vin.

### Séparation :

Le vin issu de l'unité HF est envoyé vers une décanteuse dans l'unité Ex1, où la matière solide, notamment la lignine, est séparée du vin avant l'étape de récupération de l'éthanol dans l'unité de séparation S. Dans cette étape de séparation, un flux contenant 31,5% poids de matière solide est produit. Étant donnée la nature de la séparation, ce flux contient encore une quantité importante de produits de fermentation, notamment de l'éthanol, piégés. La perte d'éthanol lors de cette étape est estimée à **20,7%.**

### Récupération de l'éthanol :

La séparation dans l'unité S se fait par distillation. Le rendement d'extraction est de 99,6%. Ainsi, le procédé permet de produire annuellement **89 707 tonnes d'éthanol**, et a une consommation spécifique d'eau de procédé de **10,2 tonnes d'eau / tonne d'éthanol.**

### Exemple 5 selon le procédé de la figure 2 (conforme à l'invention)

Dans cet exemple, on présente un procédé de production d'éthanol à partir de paille avec les caractéristiques suivantes:
Charge: Paille de blé, débit 89,60 tonne / heure, composition moyenne :

| | % (base sèche) |
|---|---|
| Cellulose | 39,1 % |
| Hémicellulose | 26,9% |
| Lignine | 17,9% |
| Autres (cendres, extractibles, etc...) | 16,1% |

### Préparation de charge et prétraitement dans l'unité P :

La paille est broyée sur une grille 50mm, puis imprégnée avec l'acide H₂SO₄ dilué à 0,7 g/litre. L'imprégnation est suivie d'une séparation solide/liquide et la liqueur d'imprégnation est recyclée, la matière sèche (MS) du solide en entrée de prétraitement est de 45% poids. Le prétraitement par explosion à la vapeur est effectué à 200°C dans une configuration continue employant un court temps de séjour. Le milieu est brusquement détendu à une pression de 1,3 atm. Le substrat prétraité est envoyé vers le réacteur d'hydrolyse enzymatique H1.

### Hydrolyse enzymatique et conversion en éthanol :

La concentration en solides à l'entrée du réacteur de liquéfaction dans l'unité H1 est de 14% poids. Pour atteindre ce niveau de dilution, 114,3 tonnes / heure d'eau de dilution sont mélangées au substrat prétraité. Cette eau de dilution contient 31,1 g d'éthanol / kg de solution et 101,7 g de pentoses / kg de solution originaires du recyclage des vinasses produites en distillation et utilisées pour le lavage du flux de matière solide obtenu à l'issue de l'opération de séparation liquide/solide Ex1 en sortie du réacteur HF.

Après 10 heures d'opération, le mélange liquéfié est envoyé dans l'unité HF, qui opère en mode SSF. Dans cette étape, les enzymes continuent à agir sur la cellulose, en même temps que le microorganisme de fermentation (*Saccharomyces cerevisiae* provenant d'une souche modifiée capable de réaliser la co-fermentation des hexoses et des pentoses) agit sur les sucres libérés. Le rendement global de la conversion de la cellulose en éthanol est de 0,35 g d'éthanol par g de cellulose introduite. La conversion des pentoses en éthanol s'élève à 0,11 g d'éthanol par g de pentose introduite. La baisse de rendement s'explique par la plus haute concentration de sucres et éthanol dans l'étape d'hydrolyse enzymatique. Le titre éthanolique du flux envoyé en séparation est de 61,0 g d'éthanol / kg de vin.

### Séparation :

Le vin issu des unités HF est envoyé vers une décanteuse dans l'unité Ex1, où la matière solide, notamment la lignine, est séparé du vin avant l'étape de récupération de l'éthanol dans l'unité de séparation S. Dans cette étape de séparation, un flux contenant 32,3% de matière solide est produit.

### Lavage du flux de matière solide :

Afin de récupérer l'éthanol piégé dans la matière solide, une étape de lavage de la matière solide est mise en oeuvre. Dans cet exemple, la totalité de l'eau envoyée comme eau de dilution à l'étape d'hydrolyse enzymatique dans l'unité H1 provient des vinasses produites en distillation dans l'unité S. Ces vinasses sont d'abord utilisées pour laver la matière solide dans l'unité de lavage L à contrecourant sous trois étages de contact. Puis les vinasses issues de l'unité L sont introduites dans l'unité H1. **Les pertes d'éthanol sont alors réduites de 92,7% par rapport aux résultats de l'exemple 4, soit 1,5% de perte globale.**

### Récupération de l'éthanol :

La séparation dans l'unité S se fait par distillation. Le rendement d'extraction est de 99,6%. Ainsi, le procédé permet de produire annuellement **114 051 tonnes d'éthanol**, et a une consommation spécifique d'eau de procédé de **0,0 tonne d'eau / tonne d'éthanol produit en régime continu.**

## Revendications

1. Procédé de production d'alcool et/ou de solvant à partir d'une charge de biomasse, dans lequel on effectue les étapes suivantes :
a) on effectue une étape de prétraitement par mise en contact et chauffage de la charge de biomasse avec de l'eau et un composé acide ou basique, de manière à obtenir un substrat prétraité,
b) on met en contact le substrat prétraité avec des enzymes cellulases et avec un flux liquide enrichi en produits de fermentation obtenu à l'étape e) de manière à obtenir un hydrolysat comportant un résidu solide et une phase liquide contenant des sucres,
c) on effectue une fermentation alcoolique de l'hydrolysat au moyen d'un microorganisme alcooligène de manière à produire un vin de fermentation comportant une matière solide et une phase liquide contenant des produits de fermentation,
d) on extrait au moins une partie de la matière solide contenue dans le vin de fermentation de manière à obtenir un flux enrichie en matière solide et un vin de fermentation appauvri en matière solide,
e) on lave le flux enrichi en matière solide avec un flux liquide de manière à obtenir ledit flux liquide enrichi en produits de fermentation, le flux liquide enrichi en produits de fermentation étant recyclé à l'étape b),
f) on effectue une étape de séparation du vin de fermentation appauvri en matière solide de manière à obtenir au moins un flux purifié comportant un alcool ou un solvant et au moins un flux de vinasse.

2. Procédé selon la revendication 1, dans lequel le flux liquide de l'étape e) est composé d'eau fraîche.

3. Procédé selon la revendication 1, dans lequel le flux liquide de l'étape e) comporte au moins une partie du flux de vinasse obtenu à l'étape f).

4. Procédé selon l'une des revendications 1 à 3, dans lequel à l'étape b), le flux liquide enrichi en produits de fermentation a un débit compris entre 50% et 1500% poids du débit de substrat prétraité.

5. Procédé selon l'une des revendications précédentes, dans lequel à l'étape e), on met en contact le flux enrichi en matière solide avec ledit flux liquide, puis on sépare le flux liquide de la matière solide.

6. Procédé selon l'une des revendications précédentes, dans lequel on réalise l'étape d) de manière à ce que ledit flux enrichi matière solide comporte entre 15% poids et 55% poids de matière solide et de manière à ce que ledit vin de fermentation appauvri en matière solide comporte moins de 15% poids de matière solide.

7. Procédé selon l'une des revendications précédentes, dans lequel on effectue l'étape b) dans un premier réacteur et on effectue l'étape c) dans un deuxième réacteur distinct du premier réacteur.

8. Procédé selon l'une des revendications 1 à 6, dans lequel on effectue simultanément l'étape b) et l'étape c) dans le même réacteur.

9. Procédé selon l'une des revendications précédentes, dans lequel les enzymes cellulases sont produites par un microorganisme choisi parmi les champignons appartenant aux genres *Trichoderma, Aspergillus, Penicillium ou Schizophyllum,* ou les bactéries anaérobies appartenant au genre *Clostridium.*

10. Procédé selon l'une des revendications précédentes, dans lequel le microorganisme alcooligène est choisi parmi les microorganismes du genre *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis, Zymomonas mobilis, Clostridium, Escherichia coli.*

11. Procédé selon l'une des revendications précédentes, dans lequel la charge de biomasse est composée par au moins l'un des éléments suivants : du bois, des plantes cultivés, des déchets lignocellulosiques agricoles, des résidus de l'industrie de transformation des matériaux lignocellulosiques.

12. Procédé selon l'une des revendications précédentes, dans lequel à l'étape a), on effectue une explosion à la vapeur de la biomasse en comprimant, puis en effectuant une détente de la biomasse en mélange avec de l'eau et un composé acide.

13. Procédé selon l'une des revendications précédentes, dans lequel à l'étape e) on met en oeuvre un organisme alcooligène produisant au moins de l'éthanol. I.

## Patentansprüche

1. Verfahren zur Erzeugung von Alkohol und/oder eines Lösungsmittels aus einer Biomassecharge, bei dem die folgenden Schritte ausgeführt werden:
a) Ausführen eines Vorbehandlungsschrittes durch Inkontaktbringen und Erhitzen der Biomassecharge mit Wasser und einer sauren oder basischen Verbindung, um ein vorbehandeltes Substrat zu erhalten,
b) Inkontaktbringen des vorbehandelten Substrats mit Zellulaseenzymen und mit einem flüssigen Strom, der mit Fermentierungsprodukten angereichert ist, der in Schritt e) erhalten wird, um ein Hydrolysat zu erhalten, das einen festen Reststoff und eine flüssige Phase, die Zucker enthält, umfasst,
c) Durchführen einer alkoholischen Fermentierung des Hydrolysats mit Hilfe eines alkoholliefernden Mikroorganismus, um Fermentierungswein zu erzeugen, umfassend einen Feststoff und eine flüssige Phase, die Fermentierungsprodukte enthält,
d) Extrahieren mindestens eines Teils des in dem Fermentierungswein enthaltenen Feststoffes, um einen mit Feststoff angereicherten Strom und an Feststoff armen Fermentierungswein zu erhalten,
e) Waschen des mit Feststoff angereicherten Stroms mit einem flüssigen Strom, um den mit Fermentierungsprodukten angereicherten flüssigen Strom zu erhalten, wobei der mit Fermentierungsprodukten angereicherte flüssige Strom in Schritt b) wiederverwertet wird,
f) Durchführen eines Trennungsschritts des an Feststoff armen Fermentierungswein, um mindestens einen gereinigten Strom zu erhalten, umfassend einen Alkohol oder ein Lösungsmittel und mindestens einen Schlempestrom.

2. Verfahren nach Anspruch 1, bei dem der flüssige Strom aus Schritt e) aus frischem Wasser besteht.

3. Verfahren nach Anspruch 1, bei dem der flüssige Strom aus Schritt e) mindestens einen Teil des in Schritt f) erhaltenen Schlempestroms umfasst.

4. Verfahren nach einem der Ansprüche 1 bis 3, bei dem in Schritt b) der mit Fermentierungsprodukten angereicherte flüssige Strom eine Menge zwischen 50 und 1500 Gew.-% der Menge des vorbehandelten Substrats aufweist.

5. Verfahren nach einem der vorhergehenden Ansprüche, bei dem in Schritt e) der mit Feststoff angereicherte Strom mit dem flüssigen Strom in Kontakt gebracht wird, dann der flüssige Strom von dem Feststoff getrennt wird.

6. Verfahren nach einem der vorhergehenden Ansprüche, bei dem Schritt d) derart ausgeführt wird, dass der mit Feststoff angereicherte Strom zwischen 15 und 55 Gew.-% Feststoff umfasst, und dass der an Feststoff arme Fermentierungswein weniger als 15 Gew.-% Feststoff umfasst.

7. Verfahren nach einem der vorhergehenden Ansprüche, bei dem der Schritt b) in einem ersten Reaktor ausgeführt wird, und der Schritt c) in einem zweiten, vom ersten Reaktor unterschiedlichen Reaktor ausgeführt wird.

8. Verfahren nach einem der Ansprüche 1 bis 6, bei dem gleichzeitig Schritt b) und Schritt c) in demselben Reaktor ausgeführt werden.

9. Verfahren nach einem der vorhergehenden Ansprüche, bei dem die Zellulaseenzyme durch einen Mikroorganismus erzeugt werden, der unter den Pilzen ausgewählt ist, der den Gattungen *Trichoderma, Aspergillus, Penicillium oder Schizophyllum* angehören, oder den anaeroben Bakterien, die der Gattung *Clostridium* angehören.

10. Verfahren nach einem der vorhergehenden Ansprüche, bei dem der alkoholliefernde Mikroorganismus ausgewählt ist unter den Mikroorganismen der Gattung *Saccharaomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis, Zymomonas mobilis, Clostridium, Escherichia coli.*

11. Verfahren nach einem der vorhergehenden Ansprüche, bei dem die Biomassecharge von mindestens einem der folgenden Elemente gebildet ist: Holz, Kulturpflanzen, Lignocellulose-Abfällen aus der Landwirtschaft, Reststoffen der Industrie zur Umwandlung der Lignocellulose-Materialien.

12. Verfahren nach einem der vorhergehenden Ansprüche, bei dem in Schritt a) eine Dampfexplosion der Biomasse durchgeführt wird, wobei komprimiert und dann eine Entspannung der Biomasse im Gemisch mit Wasser und einer sauren Verbindung durchgeführt wird.

13. Verfahren nach einem der vorhergehenden Ansprüche, bei dem in Schritt e) ein alkoholliefernder Organismus, der mindestens Ethanol erzeugt, eingesetzt wird.

## Claims

1. Process for the production of alcohol and/or solvent from a biomass feedstock, in which the following stages are carried out:
a) A pretreatment stage is carried out by heating and bringing the biomass feedstock into contact with water and an acid or base compound in such a way as to obtain a pretreated substrate,
b) The pretreated substrate is brought into contact with cellulase enzymes and with a liquid stream enriched with fermentation products and obtained in stage e) in such a way as to obtain a hydrolyzate comprising a solid residue and a liquid phase containing sugars,
c) An alcoholic fermentation of the hydrolyzate by means of an alcohologenic microorganism is carried out in such a way as to produce a fermentation wine comprising a solid material and a liquid phase containing fermentation products,
d) At least a portion of the solid material contained in the fermentation wine is extracted in such a way as to obtain a stream that is enriched with solid material and a fermentation wine that is low in solid material,
e) The stream that is enriched with solid material is washed with a liquid stream in such a way as to obtain said liquid stream that is enriched with fermentation products, with the liquid stream that is enriched with fermentation products being recycled in stage b),
f) A stage for separation of the fermentation wine that is low in solid material is carried out in such a way as to obtain at least one purified stream comprising an alcohol or a solvent and at least one vinasse stream.

2. Process according to Claim 1, in which the liquid stream from stage e) consists of fresh water.

3. Process according to Claim 1, in which the liquid stream from stage e) comprises at least a portion of the vinasse stream obtained in stage f).

4. Process according to one of Claims 1 to 3, in which in stage b), the liquid stream that is enriched with fermentation products has a flow rate of between 50% and 1,500% by weight of the flow rate of pretreated substrate.

5. Process according to one of the preceding claims, in which in stage e), the stream that is enriched with solid material is brought into contact with said liquid stream, and then the liquid stream is separated from the solid material.

6. Process according to one of the preceding claims, in which stage d) is carried out in such a way that said stream that is enriched with solid material comprises between 15% by weight and 55% by weight of solid material and in such a way that said fermentation wine that is low in solid material comprises less than 15% by weight of solid material.

7. Process according to one of the preceding claims, in which stage b) is carried out in a first reactor, and stage c) is carried out in a second reactor that is separate from the first reactor.

8. Process according to one of Claims 1 to 6, in which stage b) and stage c) are carried out simultaneously in the same reactor.

9. Process according to one of the preceding claims, in which the cellulase enzymes are produced by a microorganism that is selected from among the mushrooms that belong to the genera *Trichoderma, Aspergillus, Penicillium,* or *Schizophyllum,* or the anaerobic bacteria that belong to the genus *Clostridium.*

10. Process according to one of the preceding claims, in which the alcohologenic microorganism is selected from among the microorganisms of the genus *Saccharomyces, Saccharomyces cerevisiae, Schizosaccharomyces pombe, Saccharomyces uvarum, Saccharomyces diastaticus, Kluyveromyces fragilis, Candida shehatae, Pichia stipitis, Pachysolen tannophilis, Zymomonas mobilis, Clostridium, Escherichia coli.*

11. Process according to one of the preceding claims, in which the biomass feedstock consists of at least one of the following elements: wood, cultivated plants, agricultural lignocellulosic waste, residues of the industry for transformation of the lignocellulosic materials.

12. Process according to one of the preceding claims, in which in stage a), a vapor explosion of the biomass is carried out by exerting compression and then carrying out pressure relief of the biomass mixed with water and an acid compound.

13. Process according to one of the preceding claims, in which in stage e), an alcohologenic organism that produces at least ethanol is used.
